(19) Europäisches Patentamt
European Patent Office
Office européen des brevets

(11) Veröffentlichungsnummer: **0 269 041 B1**

(12) **EUROPÄISCHE PATENTSCHRIFT**

(45) Veröffentlichungstag der Patentschrift: **10.04.91**

(51) Int. Cl.$^5$: **C07C 215/04**, C07C 211/34, C07C 205/06, C07D 295/03, A01N 33/04

(21) Anmeldenummer: **87117207.8**

(22) Anmeldetag: **23.11.87**

Die Akte enthält technische Angaben, die nach dem Eingang der Anmeldung eingereicht wurden und die nicht in dieser Patentschrift enthalten sind.

# (54) Fungizide Cyclohexylamine.

(30) Priorität: **25.11.86 DE 3640247**

(43) Veröffentlichungstag der Anmeldung:
**01.06.88 Patentblatt 88/22**

(45) Bekanntmachung des Hinweises auf die Patenterteilung:
**10.04.91 Patentblatt 91/15**

(84) Benannte Vertragsstaaten:
**AT BE CH DE ES FR GB GR IT LI NL SE**

(56) Entgegenhaltungen:
**EP-A- 0 134 499**
**DE-C- 1 214 471**
**US-A- 3 697 594**
**EP-A- 0 259 977**
**US-A- 2 511 028**
**US-A- 3 981 766**

(73) Patentinhaber: **BASF Aktiengesellschaft**
**Carl-Bosch-Strasse 38**
**W-6700 Ludwigshafen(DE)**

(72) Erfinder: **Zipperer, Bernhard, Dr.**
**Am Herrgottsacker 6**
**W-6716 Dirmstein(DE)**
Erfinder: **Buschmann, Ernst, Dr.**
**Georg-Ludwig-Krebs-Strasse 10**
**W-6700 Ludwigshafen(DE)**
Erfinder: **Goetz, Norbert, Dr.**
**Schoefferstrasse 25**
**W-6520 Worms 1(DE)**
Erfinder: **Schirmer, Ulrich, Dr.**
**Berghalde 79**
**W-6900 Heidelberg(DE)**
Erfinder: **Ammermann, Eberhard, Dr.**
**Sachsenstrasse 3**
**W-6700 Ludwigshafen(DE)**
Erfinder: **Pommer, Ernst-Heinrich, Dr.**
**Berliner Platz 7**
**W-6703 Limburgerhof(DE)**

**EP 0 269 041 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft 4-substituierte Cyclohexylamine sowie Verfahren zu ihrer Herstellung, ihre Verwendung als Fungizide, fungizide Mittel sowie Verfahren zur Bekämpfung von Schadpilzen mit diesen Wirkstoffen.

Die Verbindung 4-trans-tert.-Butyl-N-benzyl-cyclohexylamin ist bekannt (J. Org. Chem. 48 (1983), 3412-3422). Jedoch ist nichts über eine fungizide Wirkung bekannt.

N-Alkylderivate des 4-tert.-Butylcyclohexylamins, wobei die Alkylreste 8 bis 12 C-Atome enthalten können, sind als Mikrobicide beschrieben (DE-OS 23 30 454).

Die Cyclohexylamine der Formel

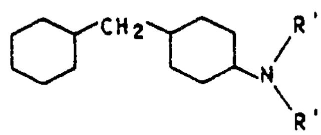

in der R' Wasserstoff oder Methyl bedeutet, sind als Fungizide bekannt (US-3 981 766).

2,6-Dimethylmorpholine mit alkylsubstituierten Cyclohexylresten am N sind als Fungizide beschrieben worden (DE-C-12 14 471). Es sind ferner substituierte Cyclohexylamine bekannt, die zum Teil eine fungizide Wirkung haben (US-A-2 511 028, EP-A-O 134 499, US-A-3 981 766, US-A-3 697 594).

Es wurde nun gefunden, daß Cyclohexylamine der Formel I

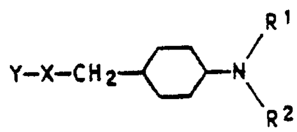

in der

| | |
|---|---|
| X | eine Einfachbindung oder eine ggf. durch eine oder mehrere Alkylgruppen mit jeweils 1 bis 5 C-Atomen substituierte Alkylenkette von 1 bis 12 C-Atomen bedeutet, in der ggf. 1 bis 4 C-Atome durch O, S oder N ersetzt sein können. |
| Y | einen Cyclohexyl-, 4-Cyclohexylcyclohexyl-, Perhydro-1- bzw. 2-naphthyl- oder Piperidylrest bedeutet, die gegebenenfalls durch eine bis drei Alkyl-, Alkoxy- oder Alkylthiogruppen mit 1 bis 12 C-Atomen oder durch ein bis drei Halogen-, Hydroxy- oder Trifluormethylgruppen substituiert sein können. |
| $R^1$ und $R^2$ | gleich oder verschieden sind und unabhängig voneinander Wasserstoff, Cycloalkyl-, Cycloalkenyl- oder Arylalkyl-Reste mit bis zu 12 C-Atomen bedeuten, die ihrerseits ggf. wiederum durch ein bis drei Alkyl-, Alkenyl-, Alkinyl-, Alkoxy- oder Alkylthioreste mit 1 bis 4 C-Atomen oder Cyclohexyl oder 4-tert.-Butylcyclohexyl substituiert sein können, oder |
| $R^1$ und $R^2$ | zusammen eine Alkylengruppe mit zwei bis sechs C-Atomen bedeuten und gemeinsam mit dem N-Atom einen Ring bilden, in dem ggf. bis zu drei C-Atome durch O, S oder N ersetzt sein können und der seinerseits ein bis drei Alkylsubstituenten mit 1 - 4 C-Atomen aufweisen kann, |
| | und ihre Säureadditionssalze, ausgenommen die Verbindungen, in denen X eine Einfachbindung, Y Cyclohexyl, $R^1$ und $R^2$ Wasserstoff bedeuten, |

eine starke fungizide Wirkung haben.

Die neuen Verbindungen können als Fungizide Verwendung finden. Die Salze der neuen Amine können beliebige anorganische oder organische Anionen enthalten, z.B. Anionen der Salzsäure, Bromwasserstoffsäure, Schwefelsäure, Salpetersäure, Essigsäure, höheren Fettsäuren, Arylsulfonsäuren.

Die neuen Amine der Formel I enthalten ggf. chirale Zentren. Sie werden im allgemeinen als Racemate und gegebenenfalls als Diastereomerengemische erhalten. Einheitliche Diastereomere lassen sich bei einigen der neuen Verbindungen beispielsweise durch Destillation, Säulenchromatographie oder aufgrund von Löslichkeitsunterschieden in reiner Form isolieren. Aus solchen gereinigten Diastereomeren kann man

mit bekannten Methoden einheitliche Racemate und Enantiomere erhalten. Alle diese Verbindungen und Gemische werden von der vorliegenden Erfindung umfaßt. Für die Anwendung der neuen Amine als Fungizide sind sowohl die einheitlichen Diastereomeren bzw. Enantiomeren wie auch deren bei der Synthese anfallenden Gemische geeignet. Bevorzugt werden die letzteren verwendet.

X bedeutet beispielsweise $C_1$-$C_4$-Alkylen, z.B. Methylen, Ethylen, 1,2-Propylen, 1,3-Propylen, 1,3-Butylen, 1,4-Butylen, 2-Alkyl-1,3-propylen, 2,2-Dialkyl-1,3-propylen, 1,5-Pentylen und beispielsweise 2-Alkyl-1,4-butylen, 2-Alkyl-1,5-pentylen, 3-Alkyl-1,5-pentylen, 1,6-Hexylen, 2-Alkyl-1,6-hexylen, 1,7-Heptylen, 1,8-Octylen, 1,9-Nonylen 1,10-Decylen, 1,11-Undecylen, 1,12-Dodecylen, Methylenoxy, Ethylenoxy, Propylenoxy, Butylenoxy, Pentylenoxy, Hexylenoxy, Heptylenoxy, Octylenoxy, Nonylenoxy, Decylenoxy, Undecylenoxy, Dodecylenoxy, Ethylamino, Propylenamino, N-$C_1$-$C_4$-Alkyl-propylenamino;

Der Rest Y bedeutet beispielsweise: Cyclohexyl, $C_1$-$C_4$-Alkylcyclohexyl, 2-, 3- oder 4-Methylcyclohexyl, 2-, 3- oder 4-Ethylcyclohexyl, 2-, 3- oder 4-Propylcyclohexyl, 2- 3- oder 4-Isopropylcyclohexyl, 2-, 3- oder 4-n-Butylcyclohexyl, 2-, 3- oder 4-tert.-Butylcyclohexyl, 4-Cyclohexylcyclohexyl, Dimethylcyclohexyl, Methylethylcyclohexyl, Methylisopropylcyclohexyl, Methyl-tert.-butylcyclohexyl, Diethylcyclohexyl, Ethylisopropylcyclohexyl, Ethyl-tert.-butylcyclohexyl, Halogencyclohexyl, 2-, 3- oder 4-Chlorcyclohexyl, 2-, 3- oder 4-Fluorcyclohexyl, 2-, 3- oder 4-Trifluormethylcyclohexyl, Hydroxycyclohexyl, $C_1$-$C_{12}$-Alkoxycyclohexyl, $C_1$-$C_{12}$-Alkylthiocyclohexyl, Perhydro-1-naphthyl, Perhydro-2-naphthyl, 1-, 2-, 3- oder 4-Piperidyl, 1-($C_1$-$C_{12}$-Alkyl)-2-, 3- oder 4-piperidyl.

$R^1$ und $R^2$ bedeuten beispielsweise Cyclopentyl, Cyclohexyl, Cyclohexylmethyl, tert.-Butylcyclohexylmethyl, Methylcyclohexyl, tert.-Butylcyclohexyl, Cycloheptyl, Cyclooctyl, Benzyl, $C_1$-$C_4$-Alkylbenzyl, Methylbenzyl, Ethylbenzyl, Dimethylbenzyl, Methylethylbenzyl, Isopropylbenzyl, tert.-Butylbenzyl oder $R^1$ und $R^2$ bedeuten zusammen mit dem Stickstoffatom, dessen Substituent sie sind, den Rest von Aziridin, Pyrrolidin, Mono-, Di- oder Trimethylpyrrolidin, Piperidin, Mono-, Di- und Trimethylpiperidin, Ethylpiperidin, Propylpiperidin, tert.-Butylpiperidin, Phenylpiperidin, Morpholin, Thiomorpholin, 2- und 3-Methylmorpholin, 2,5-Dimethylmorpholin, 2,6-Dimethylmorpholin (cis/trans oder cis oder trans), 2,6-Dimethylthiomorpholin, Piperazin, Methyl-, Ethyl-, Propyl- oder tert.-Butylpiperazin oder Hexamethylenimin.

Bevorzugt werden die Verbindungen, in denen X eine Methylen- oder 1,2-Ethylengruppe, Y Cyclohexyl oder Perhydro-Naphthyl und $R^1$ und $R^2$ Wasserstoff bedeuten.

Sekundäre und tertiäre Amine der Formel I lassen sich aus den entsprechenden primären Aminen der Formel II z.B. durch schrittweise Alkylierung herstellen.

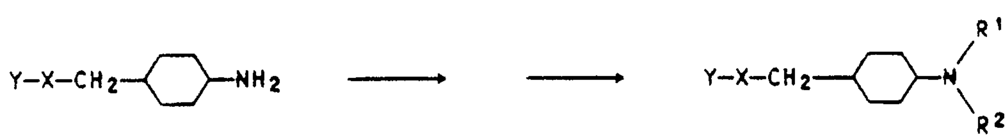

II I

Diese Alkylierungen können z.B. erfolgen

a) mit Verbindungen der Formeln III bzw. IV

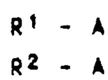 III 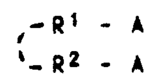 IV,

in welchen $R^1$ und $R^2$ die obengenannten Bedeutungen mit Ausnahme von Wasserstoff haben und A für eine nucleophil verdrängbare Abgangsgruppe, beispielsweise Chlor, Brom, Alkyl- oder Arylsulfonyl, steht. Die Verbindungen III bzw. IV sind bekannt und vielfach im Handel erhältlich. Als Lösungs- oder Verdünnungsmittel können für die Umsetzung beispielsweise Halogenkohlenwasserstoffe, insbesondere Chlorkohlenwasserstoffe, z.B. Dichlormethan, Trichlormethan, Tetrachlormethan, 1,2-Dichlorethan, 1,1,1- oder 1,1,2-Trichlorethan, Trichlorethylen, Chlorbenzol, Chlortoluole, Dichlorbenzole oder Ether, z.B. Diethylether, Methyl-tert.-butylether, Di-isopropylether, Di-isobutylether, Di-n-butylether, Ethylenglykoldimethylether, Tetrahydrofuran, Dioxan verwendet werden. Es können auch polare Lösungsmittel wie Aceton, Methylethylketon, Acetonitril, Dimethylformamid, N-Methylpyrrolidon, Ethylacetat, Nitromethan, Dimethylsulfoxid verwendet werden. Anstelle eines Lösungsmittels können auch die Alkylierungsmittel der Formeln III bzw. IV im Überschuß eingesetzt werden. Als Hilfsbasen (Säureakzeptoren) für die

Umsetzung zu Verbindungen der Formel I können alle üblichen Säurebindemittel verwendet werden. Hierzu gehören vorzugsweise tertiäre Amine, Alkali- oder Erdalkalioxide, -hydroxide oder -salze, sowie basische Ionenaustauscher. Beispielsweise kommen als basische Verbindungen in Frage: Kaliumhydroxid, Natriumhydroxid, Calciumhydroxid, Bariumhydroxid, Calciumoxid, Bariumoxid, Magnesiumoxid, Kaliumcarbonat, Natriumcarbonat, Natriumhydrogencarbonat, Natriumacetat, Triethylamin, Tri-n-propylamin, Tri-n-butylamin, Ethyl-diisopropylamin, N,N-Dimethylcyclohexylamin, Pyridin, Methylpyridine, Chinolin, Isochinolin, 2,6-Lutidin, 2,4,6-Kollidin.

Die Alkylierungen können z.B. auch erfolgen

b) mit Aldehyden oder Ketonen der allgemeinen Formeln

$R^8$—CHO bzw. 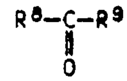

(XII) (XII)

in denen die Reste $R^8$, $R^9$ den Resten $R^1$, $R^2$ entsprechen, mit der Maßgabe, daß sie ein C-Atom weniger als $R^1$, $R^2$ aufweisen, also bis zu 11 C-Atome aufweisendes Cycloalkyl, Cycloalkenyl oder Arylalkyl bedeuten, die ihrerseits ggf. wiederum durch ein bis drei geradkettige oder verzweigte Alkyl-, Alkenyl-, Alkinyl-, Alkoxy- oder alkylthioreste mit 1 bis 4 C-Atomen oder Cyclohexyl oder 4-tert.-Butylcyclohexyl substituiert sein können.

Die Aldehyde bzw. Ketone werden in Gegenwart eines Reduktionsmittels zweckmäßig in einem Lösungsmittel ggf. in Gegenwart eines Katalysators mit den entsprechenden primären bzw. sekundären Aminen umgesetzt, können aber auch zunächst mit den entsprechenden primären bzw. sekundären Aminen zu Iminen bzw. Enaminen umgesetzt werden, welche dann anschließend, ggf. in Gegenwart eines Katalysators zu den Aminen der Formel I reduziert werden.

Die Aldehyde bzw. Ketone sind bekannte Verbindungen und vielfach im Handel erhältlich.

Als Reduktionsmittel bei der Durchführung des erfindungsgemäßen Verfahrens eignen sich z.B. komplexe Hydride, vorzugsweise Lithiumborhydrid, Natriumborhydrid, Natriumcyanborhydrid, Lithiumaluminiumhydrid, Lithium-tri-tert.-butoxy-aluminiumhydrid, Lithium-tri-(2-methyl-but-2-yl)borhydrid. Ferner eignen sich Wasserstoff in Gegenwart eines Katalysators, z.B. eines Edelmetalls, vorzugsweisie Palladium, das ggf. auf ein Trägermaterial, z.B. Aktivkohle, aufgebracht wurde und Ameisensäure (Leuckart-Wallach-Reaktion; vgl. Methoden der Org. Chemie (Houben-Weyl), Bd. XI/1 (1957), S. 648 ff).

Als Verdünnungsmittel können je nach Art des verwendeten Reduktionsmittels aprotische oder auch protische Lösungsmittel dienen, z.B. Ether, vorzugsweise Diethylether, Tetrahydrofuran, Dioxan, Ethylenglykoldimethylether, Ester, vorzugsweise Ethylacetat oder Alkohole, vorzugsweise Methanol, Ethanol, Propanole und Butanole.

Die zur Alkylierung verwendeten neuen primären Amine II können z.B. dadurch hergestellt werden, daß man Phenylnitroverbindungen der Formel V

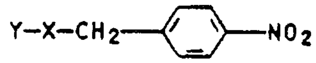

(V)

zunächst nach bekannten Methoden (vgl. z.B. Methoden der Organischen Chemie (Houben-Weyl), Band XI/1 (1957), S. 360 - 515) zu den entsprechenden Anilinen reduziert und diese dann nach bekanntem Verfahren (vgl. EP 53 818) mit Wasserstoff in Gegenwart eines Katalysators unter Druck hydriert.

Die Zwischenprodukte der Formel V sind neu und können nach verschiedenen Verfahren hergestellt werden, indem man z.B.

a) Phosphoniumsalze der Formel VI

$$Y{-}X{-}P^{\oplus}(C_6H_5)_3{-}Hal^{\ominus} \qquad VI$$

in der X und Y die die oben genannte Bedeutung haben und $Hal^{\ominus}$ für Chlorid oder Bromid steht, mit 4-Nitrobenzaldehyd in Gegenwart einer Base in einem Lösungsmittel umsetzt.

Die Phosphoniumsalze (VI) sind durch Umsetzung der entsprechenden Halogenide Y-X-Hal mit Triphenylphosphan in einem Lösungsmittel, beispielsweise Benzol, Toluol, Xylol bei erhöhter Temperatur, vorzugsweise 80 bis 130 °C, zugänglich.

Für die Umsetzung der Phosphoniumsalze (VI) mit 4-Nitrobenzaldehyd eignen sich als Basen z.B. Alkalihydroxide, -alkoholate, -hydride und metallorganische Verbindungen, beispielsweise Natriumhydroxid, Kaliumhydroxid, Natriummethylat, Natriumethylat, Kalium-tert.-butylat, Natriumhydrid, Kaliumhydrid, n-Butyllithium, sec.-Butyllithium, tert.-Butyllithium, Phenyllithium, Methyllithium, Lithiumdiisopropylamid. Als Lösungsmittel kommen je nach Art der verwendeten Base protische oder aprotische Solventien in Frage, z.B. Alkohole wie Methanol, Ethanol, tert.-Butanol, Ether, beispielsweise Diethylether, Tetrahydrofuran, Dioxan, Ethylenglykoldimethylether, ggf. auch Benzol oder Dimethylsulfoxid (vgl. Methoden der Organischen Chemie (Houben-Weyl) Bd. V/ 1 (1972), S. 383 ff).

Zwischenprodukte der Formel V lassen sich auch herstellen, indem man

b) Aldehyde der Formel VII

$$Y-(CH=CH)_n-CHO \qquad VII$$

in der Y die oben genannte Bedeutung hat und n die Werte 0 oder 1 annehmen kann, in Gegenwart eines Katalysators mit 4-Nitroacetophenon umsetzt.

Als Katalysatoren für diese Kondensation kommen sowohl saure als auch basische Stoffe in Frage. Beispielsweise eignen sich als Säuren Chlorwasserstoff, Schwefelsäure, Borsäure, Essigsäure, Arylsulfonsäuren, als Basen Alkali- und Erdalkali-oxide, -hydroxide und -alkoholate wie z.B. Natriumhydroxid, Bariumhydroxid, Natriummethylat, Natriumethylat und Kalium-tert.-butylat.

Die Aldehyde VII sind bekannte Verbindungen und vielfach im Handel erhältlich.

Die Kondensationen können mit oder ohne Verdünnungsmittel durchgeführt werden.

Als Verdünnungsmittel eignen sich protische und aprotische Lösungsmittel, beispielsweise Alkohole, z.B. Methanol, Ethanol, Propanole, Butanole, ggf. auch im Gemisch mit Wasser, Kohlenwasserstoffe, z.B. Benzol, Toluol, Xylole, Ether, z.B. Dimethylether, Tetrahydrofuran, Dioxan, Sulfoxide, z.B. Dimethylsulfoxid`

Die Reaktionen können je nach Art des verwendeten Katalysators in der Kälte, vorzugsweise bei -10 bis +30 °C oder in der Wärme, bevorzugt bei +40 bis +150 °C durchgeführt und ggf. durch Entfernen des gebildeten Reaktionswassers beschleunigt werden.

Zwischenprodukte der Formel V lassen sich auch herstellen, indem man

c) Verbindungen der Formel X

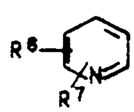

(X)

in der $R^6$ und $R^7$ die für Y definierten Substituenten des Piperidinrestes bedeuten, mit Verbindungen der Formel XI

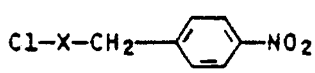

(XI)

in der X die oben genannte Bedeutung hat, in einem Lösungsmittel umsetzt und die Reaktionsprodukte hydriert.

Die Pyridinderivate der Formel X sind bekannt und vielfach im Handel erhältlich. Es handelt sich dabei beispielsweise um Pyridin, 2-, 3- und 4-Picolin, 2-, 3- und 4-Ethylpyridin, 2-, 3- und 4-Propylpyridin, 2-, 3- und 4-Isopropylpyridin, 2-, 3- und 4-Phenylpyridin, 4-tert.-Butylpyridin, 2,6-Lutidin, 2,4,6-Kollidin.

Die ω-(4-Nitrophenyl)alkylchloride der Formel XI sind bekannt. Es handelt sich beispielsweise um 4-Nitrobenzylchlorid, 2-(4-Nitrophenyl)ethylchlorid, 3-(4-Nitrophenyl)propylchlorid, 2-(4-Nitrophenyl)-propylchlorid, 4-(4-Nitrophenyl)butylchlorid.

Als Lösungsmittel für die Umsetzung der Verbindungen X und XI kommen sowohl protische als auch

aprotische Lösungsmittel hoher Polarität in Frage, z. B. Alkohole, insbesondere Methanol, Ethanol, Propanole, Butanole, Ester, insbesondere Essigsäuremethylester, Essigsäureethylester, Nitrile, beispielsweise Acetonitril, Propionitril, Amide, z. B. Dimethylformamid, N-Methylformanilid, N-Methylpyrrolidon, Nitroverbindungen, vorzugsweise Nitromethan, 1- bzw. 2-Nitropropan, Nitrobenzol. Die Umsetzungen werden im allgemeinen bei Temperaturen zwischen +20 und +200° C, drucklos oder unter Druck, durchgeführt.

Zu den Zwischenprodukten der Formel V ähnliche Verbindungen lassen sich auch herstellen, indem man aromatische Verbindungen der Formel VIII

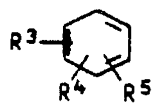

VIII

in der $R^3$, $R^4$, $R^5$ die für Y definierten Substituenten des Arylrestes bedeuten, in Gegenwart einer Lewis-Säure, ggf. in einem Lösungs- bzw. Verdünnungsmittel mit 4-Nitrobenzoylchlorid umsetzt und die gebildeten Diarylketone anschließend reduziert, z.B. mit Hydrazin/Kaliumhydroxid (Wolff-Kizner Reduktion, vgl. D. Todd, Org. Reactions 4 (1948), 378 - 422) oder mit Metallen oder Metall-Legierungen, beispielsweise Zinkamalgam (Clemmensen-Reduktion, vgl. E.L. Martin, Org. Reactions 1 (1942), 155 -209) reduziert.

Die aromatischen Verbindungen der Formel VIII sind bekannt und vielfach im Handel erhältich. Unter VIII versteht man beispielsweise Kohlenwasserstoffe wie z.B. Benzol, Toluol, Xylole, Ethylbenzol, Cumol, tert.-Butylbenzol, Biphenyl, Naphthalin, Phenanthren, Anthracen, Halogenkohlenwasserstoffe, z.B. Fluorbenzol, Chlorbenzol, Brombenzol, Chlornaphthaline, Arylether, z.B. Anisol, Phenetol, tert. Butoxybenzol, Methoxynaphthaline, Diphenylether.

Als Lewis-Säuren eignen sich für die Reaktion von Verbindungen der Formel VIII mit 4-Nitrobenzoylchlorid wasserfreie Halogenide, beispielsweise Bortrichlorid, Aluminiumtrichlorid, Phosphortrichlorid, Zinkchlorid.

Die Reaktion von Verbindungen der Formel VIII mit 4-Nitrobenzoylchlorid kann ggf. in einem Verdünnungsmittel durchgeführt werden. Hierfür eignen sich beispielsweise Halogenkohlenwasserstoffe z.B. Dichlormethan, 1,2-Dichlorethan oder andere unter den Reaktionsbedingungen inerte Lösungsmittel, beispielsweise Schwefelkohlenstoff und Nitrobenzol.

Zu den Zwischenprodukten der Formel V ähnliche Verbindungen lassen sich auch herstellen, indem man
Verbindungen der Formel IX

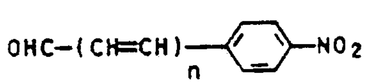

(IX)

in der n die Werte 0 oder 1 annehmen kann mit 2- bzw. 4-Methylpyridin in Gegenwart eines Katalysators kondensiert.

Die Aldehyde der Formel IX sind bekannt und im Handel erhältlich. Die Reaktion mit 2- bzw. 4-Methylpyridin wird zweckmäßig in Gegenwart eines sauren Katalysators, z. B. wasserfreiem Zinkchlorid oder Acetanhydrid bei erhöhter Temperatur, vorzugsweise 140 - 230° C, durchgeführt

Vorschrift 1

4-Methyl-1-(4-nitrobenzyl)pyridiniumchlorid

Eine Lösung von 34,4 g (0,2 mol) 4-Nitrobenzylchlorid und 27,9 g (0,3 mol) 4-Methylpyridin in 200 ml Acetonitril wird 8 Std. am Rückflußerhitzt. Man engt auf etwa die Hälfte des Volumens ein, kühlt auf 0° C ab, saugt den ausgefallenen Niederschlag ab und wäscht gut mit Ether nach. Nach Trocknen i. Vak. 37,6 g (71 % d. Th.) der Titelverbindung, Schmp. 220 - 222° C.

Vorschrift 2

2-(4-Nitro-trans-styryl)pyridin

Die Lösung von 46,5 g (0,5 mol) 2-Methylpyridin und 75,5 g (0,5 mol) 4-Nitrobenzaldehyd in ca. 100 g Acetanhydrid (ca. 1 mol) wird 6 Std. am Rückfluß erhitzt, dann vorsichtig auf Eiswasser gegossen. Unter Rühren wird mit gesättigter. wäßriger Natriumhydrogencarbonat-Lösung neutralisiert. Der abgesaugte Niederschlag wird mit Wasser gewaschen und aus Ethanol umkristallisiert. 66 g (63 % d. Th.) rotbraune Kristalle vom Schmp. 136 -139 °C.

Vorschrift 3

4-Nitro-4' -phenyl-benzophenon

Zur Suspension von 160 g (1,2 mol) wasserfreiem Aluminumchlorid in 400 ml 1,2-Dichlorethan tropft man bei 0 °C nacheinander die Lösungen von 147 g (1,05 mol) 4-Nitrobenzoylchlorid in 250 ml 1,2-Dichlorethan und von 154 g (1,0 mol) Biphenyl in 200 ml 1,2-Dichlorethan. Man läßt auf Raumtemp. kommen, rührt dann 2 Std. bei 50 °C und gießt vorsichtig auf Eiswasser. Der abgesaugte Niederschlag wird gut gewaschen und aus Ethanol umkristallisiert. 230 g (89 % d. Th.) braune Kristalle vom Schmp. 174 -176 °C.

Vorschrift 4

E-(3-Methylstyryl)-4-nitrophenyl-keton

Die Lösung von 76 g (0,63 mol) 3-Methylbenzaldehyd und 104,5 g (0,63 mol) 4-Nitroacetophenon in 1 l Xylol wird mit 10 g Borsäure versetzt und 8 Std. am Wasserabscheider unter Rückfluß gekocht. Nach Zugabe von weiteren 5 g Borsäure wird erneut 8 Std. gekocht. Dieser Vorgang wird so oft wiederholt, bis die theoretisch berechnete Wassermenge abgeschieden ist (2 - 3 mal). Man saugt die gekühlte Reaktionsmischung ab, wäscht den Rückstand mit Toluol und trocknet ihn i. Vak. 131 g (77 % d. Th.) gelbbraunes Pulver vom Schmp. 130 - 132 °C.

Vorschrift 5

4-[3-(3-Methylphenyl)propyl]anilin

Die Lösung von 130 g (0,49 mol) E-(3-Methylstyryl)-4-nitrophenyl-keton in 1,3 l Eisessig wird mit 46 g (0,47 mol) konz. Schwefelsäure und 8 g Palladium auf 80 % Aktivkohle versetzt und mit Stickstoff gesättigt. Dann wird bei Normaldruck unter Rühren Wasserstoff eingegast. Nachdem ca. 30 l Wasserstoff aufgenommen wurden, erwärmt man auf 50 - 70 °C und hydriert bis zum Verbrauch der theoretisch berechneten Wasserstoffmenge (ca. 66 l). Man saugt über Kieselgur ab, engt zur Trockne ein und verrührt den Rückstand mit 1 l Eiswasser, 200 ml 50-proz. Natronlauge und 800 ml Methyl-tert.-butylether. Die wäßrige Phase wird 3 mal mit je 250 ml Methyl-tert.-butylether gewaschen, die vereinigten organ. Phasen werden je 1 mal mit 250 ml Wasser bzw. gesätt. NaCl-Lösung gewaschen, getrocknet ($Na_2SO_4$) und i. Vak. eingeengt. 114 g braunes Öl, lt. GC (Gaschromatogramm) ca. 95 prozentig (entspr. 98 % d. Th.). Siedepunkt 158 -163 °C/0,3.mm.

Beispiel 1

cis,trans-4-[3-(3-Methylcyclohexyl)propyl]cyclohexylamin

Die Lösung von 63 g (0,28 mol) 4-[3-(3-Methylphenyl)propyl]anilin in 100 ml Dioxan wird mit 0,5 g Rutheniumdioxid versetzt und in einem 300 ml-Rührautoklaven bei 140 °C und 300 bar $H_2$ bis zur Druckkonstanz hydriert (ca. 3 h). Der Austrag wird über Kieselgur abgesaugt und i. Vak. eingeengt, der Rückstand fraktioniert destilliert. 47 g (71 % d. Th.) farbloses Öl. Sdp. 100 -116 °C/0,2 mm, bestehend aus ca. 30 % cis- und 70 % trans-Isomer (Verbindung Nr. 64).

Beispiel 2

N-(4-tert.-Butylbenzyl)-4-[3-(3-methylcyclohexyl)propyl]cyclohexylamin

Die Lösung von 11,9 g (0,05 mol) 4-[3-(3-Methylcyclohexyl)propyl]cyclohexylamin in 50 ml wasserfreiem Methanol wird mit einer methanolischen HCl-Lösung auf pH 5 -6 angesäuert, dann mit 8,1 g (0,05 mol) 4-tert.-Butylbenzaldehyd und 15 g trockenem Molekularsieb (3 Å, Grace Typ0 564) versetzt und auf 0 °C gekühlt. Man gibt unter Rühren portionsweise 1,6 g (0,025 mol) Natriumcyanborhydrid zu, erwärmt auf Raumtemperatur und rührt 24 Std. Danach wird mit konz. HCl angesäuert, entstandener Cyanwasserstoff mit Stickstoff ausgetrieben, filtriert und mit verd. NaOH alkalisch gestellt. Man exthahiert mehrfach mit Dichlormethan, trocknet die organ. Phase über $MgSO_4$, engt ein und destilliert. 9,5 g (56 % d. Th. ) gelbes Öl vom Sdp. 236 - 242 °C/0,4 mm (Verbindung. Nr. 65).

Die folgenden Verbindungen können in entsprechender Weise hergestellt werden:

Tabelle 1

| Beispiel Nr. | X | Y | $R^1$ | $R^2$ | Phys. Daten Kp oder Fp) |
|---|---|---|---|---|---|
| 2 | - | Cyclohexyl | $-CH_2CH(CH_3)OCH(CH_3)CH_2-$ | | |
| 3 | | Cyclohexyl | $-CH_2CH_2-$ | | |
| 4 | - | Cyclohexyl | $-CH_2(CH_2)_2CH_2-$ | | |
| 5 | - | Cyclohexyl | $-CH_2(CH_2)_3CH_2-$ | | |
| 6 | - | 4-Methylcyclohexyl | H | H | |
| 7 | - | 4-Methylcyclohexyl | H | $-CH_2(p-C_6H_4)C(CH_3)_3$ | |
| 8 | - | 4-Chlorcyclohexyl | H | H | |
| 9 | - | 4-Chlorcyclohexyl | H | $-CH_2(p-C_6H_4)C(CH_3)_3$ | |
| 10 | - | 4-Fluorcyclohexyl | H | H | |
| 11 | - | 4-Fluorcyclohexyl | H | $-CH_2(p-C_6H_4)C(CH_3)_3$ | |
| 12 | - | 1-Piperidyl | H | H | |
| 13 | - | 1-Piperidyl | H | $-CH_2(p-C_6H_4)C(CH_3)_3$ | |
| 14 | - | Perhydro-2-naphthyl | H | H | 127-131°C/ 0,3 mm |
| 15 | $CH_2$ | Cyclohexyl | H | H | |
| 17 | $CH_2$ | Cyclohexyl | H | $-CH_2(p-C_6H_4)C(CH_3)_3$ | |
| 18 | $CH_2$ | 4-Methylcyclohexyl | H | H | 116-122°C/ 0,5 mm |
| 19 | $CH_2$ | 4-Methylcyclohexyl | | $-CH_2(p-C_6H_4)C(CH_3)_3$ | |
| 20 | $CH_2$ | 4-Trifluormethylcyclohexyl | H | H | |

Tabelle 1 (Fortsetzung)

| Beispiel Nr. | X | Y | $R^1$ | $R^2$ | Phys. Daten Kp oder Fp) |
|---|---|---|---|---|---|
| 21 | $CH_2$ | 4-Trifluormethylcyclohexyl | H | $-CH_2(p-C_6H_4-)C(CH_3)_3$ | |
| 22 | $CH_2$ | 4-tert.-Butylcyclohexyl | H | H | 132-140°C/ |
| | | | H | | 0,2 mm |
| 23 | $CH_2$ | 4-tert.-Butylcyclohexyl | $-CH_2CH(CH_3)OCH(CH_3)CH_2-$ | | 233-240°C/ 0,4 mm |
| 24 | $CH_2$ | 4-tert.-Butylcyclohexyl | H | $-CH_2(p-C_6H_4)C(CH_3)_3$ | |
| 25 | $CH_2$ | Perhydro-1-naphthyl | H | H | 152-154°C/ 0,3 mm |
| 26 | $CH_2$ | Perhydro-1-naphthyl | $-CH_2CH(CH_3)OCH(CH_3)CH_2-$ | | 200-208°C/ 0,4 mm |
| 27 | $CH_2$ | 4-Fluorcyclohexyl | H | H | |
| 28 | $CH_2$ | 4-Fluorcyclohexyl | H | $-CH_2(p-C_6H_4)C(CH_3)$ | |
| 29 | $CH_2$ | 4-Chlorcyclohexyl | H | H | |
| 30 | $CH_2$ | 4-Chlorcyclohexyl | H | $-CH_2(p-C_6H_4)C(CH_3)$ | |
| 31 | $CH_2$ | 4-Chlorcyclohexyl | $-CH_2(CH_2)_3CH_2-$ | | |
| 32 | $CH_2$ | 3-Chlorcyclohexyl | H | H | |
| 33 | $CH_2$ | 3-Chlorcyclohexyl | H | $-CH_2(p-C_6H_4)C(CH_3)$ | |
| 34 | $CH_2$ | 2-Chlorcyclohexyl | H | H | |
| 35 | $CH_2$ | 2-Chlorcyclohexyl | H | $-CH_2(p-C_6H_4)C(CH_3)_3$ | |

Tabelle 1 (Fortsetzung)

| Beispiel Nr. | X | Y | $R^1$ | $R^2$ | Phys. Daten Kp oder Fp) |
|---|---|---|---|---|---|
| 40 | $CH_2$ | 1-Piperidyl | H | H | |
| 41 | $CH_2$ | 1-Piperidyl | H | $-CH_2(p-C_6H_4)C(CH_3)_3$ | |
| 42 | $CH_2$ | 2-Piperidyl | H | H | |
| 43 | $CH_2$ | 2-(1-Methylpiperidyl) | H | H | |
| 44 | $CH_2$ | 2-(1-Methylpiperidyl) | H | $-CH_2(p-C_6H_4)C(CH_3)_3$ | |
| 45 | $CH_2$ | 2-(1-Methylpiperidyl) | H | H | |
| 46 | $CH_2$ | 3-Piperidyl | H | H | |
| 47 | $CH_2$ | 3-(1-Methylpiperidyl) | H | H | |
| 48 | $CH_2$ | 3-(1-Methylpiperidyl) | H | $-CH_2(p-C_6H_4)C(CH_3)_3$ | |
| 49 | $CH_2$ | 4-Piperidyl | H | H | |
| 50 | $CH_2$ | 4-(1-Methylpiperidyl) | H | H | |
| 51 | $CH_2$ | 4-(1-Methylpiperidyl) | H | $-CH_2(p-C_6H_4)C(CH_3)_3$ | |
| 52 | $CH_2$ | 4-(1-Propylpiperidyl) | H | H | |
| 53 | $(CH_2)_2$ | Cyclohexyl | H | H | |
| 54 | $(CH_2)_2$ | Cyclohexyl | H | $-CH_2(p-C_6H_4)C(CH_3)_3$ | |
| 56 | $(CH_2)_2$ | Cyclohexyl | $-CH_2CH(CH_3)OCH(CH_3)CH_2-$ | | |
| 57 | $(CH_2)_2$ | Cyclohexyl | $-CH_2CH_2-$ | | |
| 58 | $(CH_2)_2$ | Cyclohexyl | $-CH_2(CH_2)_2CH_2-$ | | |

Tabelle 1 (Fortsetzung)

| Beispiel Nr. | X | Y | $R^1$ | $R^2$ | Phys. Daten Kp oder Fp) |
|---|---|---|---|---|---|
| 59 | $(CH_2)_2$ | 4-Methylcyclohexyl | H | H | |
| 61 | $(CH_2)_2$ | 4-Methylcyclohexyl | H | $-CH_2(p-C_6H_4)C(CH_3)_3$ | |
| 62 | $(CH_2)_2$ | 4-Methylcyclohexyl | | $-CH_2(CH_2)_2CH_2-$ | |
| 63 | $(CH_2)_2$ | 4-Methylcyclohexyl | | $-CH_2CH(CH_3)OCH(CH_3)CH_2-$ | 190-194°C/ 1,0 mm |
| 64 | $(CH_2)_2$ | 3-Methylcyclohexyl | H | H | |
| 65 | $(CH_2)_2$ | 3-Methylcyclohexyl | H | $-CH_2(p-C_6H_4)C(CH_3)_3$ | |
| 66 | $(CH_2)_2$ | 2-Methylcyclohexyl | H | H | |
| 68 | $(CH_2)_2$ | 2-Methylcyclohexyl | | $-CH_2CH(CH_3)OCH(CH_3)CH_2-$ | |
| 69 | $(CH_2)_2$ | 3,4-Dimethylcyclohexyl | H | H | 144-145°C/ 0,8 mm |
| 70 | $(CH_2)_2$ | 3,4-Dimethylcyclohexyl | | $-CH_2CH(CH_3)OCH(CH_3)CH_2-$ | 195-197°C/ 0,5 mm |
| 71 | $(CH_2)_2$ | 3,4-Dimethylcyclohexyl | | $-CH_2CH(CH_3)OCH(CH_3)CH_2-$ | |
| 72 | $(CH_2)_2$ | 4-Ethylcyclohexyl | H | H | 136-144°C/ 0,3 mm |
| 74 | $(CH_2)_2$ | 4-Ethylcyclohexyl | H | $-CH_2(p-C_6H_4)C(CH_3)_3$ | |
| 75 | $(CH_2)_2$ | 4-Ethylcyclohexyl | | $-CH_2CH(CH_3)(OCH(CH_3)CH_2$ | 200°C/ 0,8 mm |

Tabelle 1 (Fortsetzung)

| Bei- spiel Nr. | X | Y | $R^1$ | $R^2$ | Phys. Daten Kp oder Fp) |
|---|---|---|---|---|---|
| 76 | $(CH_2)_2$ | 4-(2-Propyl)cyclohexyl | H | H | 132-134°C/ 0,3 mm |
| 77 | $(CH_2)_2$ | 4-(2-Propyl)cyclohexyl | H | $-CH_2(p-C_6H_4)C(CH_3)_3$ | |
| 78 | $(CH_2)_2$ | 4-(2-Propyl)cyclohexyl | $-CH_2CH(CH_3)OCH(CH_3)CH_2-$ | | 197-200°C/ 0,4 mm |
| 79 | $(CH_2)_2$ | 4-tert.-Butylcyclohexyl | H | H | |
| 80 | $(CH_2)_2$ | 4-tert.-Butylcyclohexyl | H | $-CH_2(p-C_6H_4)C(CH_3)_3$ | |
| 81 | $(CH_2)_2$ | 4-tert.-Butylcyclohexyl | $-CH_2(CH_2)_2CH_2-$ | | |
| 82 | $(CH_2)_2$ | 4-Cyclohexylcyclohexyl | H | H | 188-190°C/ 0,4 mm |
| 83 | $(CH_2)_2$ | 4-Cyclohexylcyclohexyl | H | $-CH_2(p-C_6H_4)C(CH_3)_3$ | |
| 84 | $(CH_2)_2$ | 4-Trifluormethylcyclohexyl | H | H | |
| 85 | $(CH_2)_2$ | 4-Trifluormethylcyclohexyl | H | $-CH_2(p-C_6H_4)C(CH_3)_3$ | |
| 86 | $(CH_2)_2$ | 4-Fluorcyclohexyl | H | H | |
| 87 | $(CH_2)_2$ | 4-Chlorcyclohexyl | H | H | |
| 88 | $(CH_2)_2$ | 4-Chlorcyclohexyl | H | $-CH_2(p-C_6H_4)C(CH_3)_3$ | |
| 90 | $(CH_2)_2$ | 4-Chlorcyclohexyl | H | H | |
| 92 | $(CH_2)_2$ | 3-Chlorcyclohexyl | H | H | |
| 93 | $(CH_2)_2$ | 3-Chlorcyclohexyl | H | $-CH_2(p-C_6H_4)C(CH_3)_3$ | |
| 94 | $(CH_2)_2$ | 2-Chlorcyclohexyl | H | H | |
| 95 | $(CH_2)_2$ | 2-Chlorcyclohexyl | H | $-CH_2(p-C_6H_4)C(CH_3)_3$ | |

Tabelle 1 (Fortsetzung)

| Beispiel Nr. | X | Y | $R^1$ | $R^2$ | Phys. Daten Kp oder Fp) |
|---|---|---|---|---|---|
| 98 | $(CH_2)_2$ | Perhydro-1-naphthyl | H | H | 150-155°C/ 0,3 mm |
| 99 | $(CH_2)_2$ | Perhydro-1-naphthyl | $-CH_2CH(CH_3)OCH(CH_3)CH_2$ | | 222-228°C/ 0,5 mm |
| 100 | $(CH_2)_2$ | Perhydro-1-naphthyl | H | H | 176-184°C/ 0,5 mm |
| 102 | $(CH_2)_2$ | Perhydro-1-naphthyl | $-CH_2CH(CH_3)OCH(CH_3)CH_2-$ | | 211-212°C/ 0,4 mm |
| 103 | $(CH_2)_2$ | 1-Piperidyl | H | H | |
| 104 | $-CH(CH_3)CH_2-$ | Cyclohexyl | H | H | 139-141°C/ 2,0 mm |
| 105 | $-CH(CH_3)CH_2-$ | 4-Methylcyclohexyl | H | H | 144-148°C/ 1,5 mm |
| 106 | $-CH(CH_3)CH_2-$ | 4-Methylcyclohexyl | H | $-CH_2(p-C_6H_4)C(CH_3$ | |
| 107 | $-CH(CH_3)CH_2-$ | 4-tert.-Butylcyclohexyl | H | H | |
| 108 | $-CH(CH_3)CH_2-$ | 4-tert.-Butylcyclohexyl | H | $-CH_2(p-C_6H_4)C(CH_3$ | |
| 109 | $-CH(CH_3)CH_2$ | 4-tert.-Butylcyclohexyl | $-CH_2CH(CH_3)OCH(CH_3)CH_2$ | | 176°C/ 0,1 mm |

Tabelle 1 (Fortsetzung)

| Beispiel Nr. | X | Y | $R^1$ | $R^2$ | Phys. Daten Kp oder Fp) |
|---|---|---|---|---|---|
| 114 | $-CH_2O-$ | Cyclohexyl | H | Cyclohexyl | |
| 115 | $-CH_2O-$ | Cyclohexyl | H | $-CH_2(p-C_6H_4)C(CH_3)_3$ | |
| 116 | $(CH_2)_3$ | Cyclohexyl | H | H | |
| 117 | $(CH_2)_3$ | Cyclohexyl | H | $-CH_2(p-C_6H_4)C(CH_3)_3$ | |
| 118 | $(CH_2)_3$ | 4-Methylcyclohexyl | H | H | |
| 119 | $(CH_2)_3$ | 4-Methylcyclohexyl | H | $-CH_2(p-C_6H_4)C(CH_3)_3$ | |
| 120 | $(CH_2)_3$ | 4-tert.-Butylcyclohexyl | H | H | |
| 121 | $(CH_2)_3$ | 4-tert.-Butylcyclohexyl | H | $-CH_2(p-C_6H_4)C(CH_3)_3$ | |
| 122 | $(CH_2)_3$ | 4-Chlorcyclohexyl | H | H | |
| 123 | $(CH_2)_3$ | 4-Chlorcyclohexyl | H | $-CH_2(p-C_6H_4)C(CH_3)_3$ | |
| 125 | $(CH_2)_3$ | 2-Piperidyl | H | H | |
| 126 | $(CH_2)_3$ | 2-(1-Methylpiperidyl) | H | H | |
| 127 | $(CH_2)_3$ | 4-Piperidyl | H | H | |
| 128 | $(CH_2)_3$ | 4-(1-Methylpiperidyl) | H | H | |
| 129 | $(CH_2)_4$ | Cyclohexyl | H | H | |
| 130 | $(CH_2)_4$ | Cyclohexyl | H | $-CH_2(p-C_6H_4)C(CH_3)_3$ | |
| 131 | $(CH_2)_4$ | 4-Methylcyclohexyl | H | H | 162°C/ 0,3 mm |
| 132 | $(CH_2)_4$ | 4-Methylcyclohexyl | H | $-CH_2(p-C_6H_4)C(CH_3)_3$ | |
| 133 | $(CH_2)_4$ | 4-Methylcyclohexyl | $-CH_2CH(CH_3)OCH(CH_3)CH_2$ | | 198-210°C/ 0,4 mm |

Tabelle 1 (Fortsetzung)

| Beispiel Nr. | X | Y | $R^1$ | $R^2$ | Phys. Daten Kp oder Fp) |
|---|---|---|---|---|---|
| 134 | $(CH_2)_4$ | 4-tert.-Butylcyclohexyl | H | H | |
| 135 | $(CH_2)_4$ | 4-tert.-Butylcyclohexyl | H | $-CH_2(p-C_6H_4)C(CH_3)_3$ | |
| 136 | $(CH_2)_2CH(C_6H_{11})CH_2$ | Cyclohexyl | H | H | |
| 137 | $-NH(CH_2)_3-$ | Cyclohexyl | H | H | |
| 138 | $-N(CH_3)(CH_2)_3-$ | Cyclohexyl | | H | |
| 139 | $-N(CH_3)(CH_2)_3-$ | Cyclohexyl | H | $-CH_2(p-C_6H_4)C(CH_3)_3$ | |
| 140 | $-N(CH_3)(CH_2)_3-$ | Cyclohexyl | | $-CH_2(CH_2)_2CH_2-$ | |
| 142* | $-[C(CH_3)_2]_2-$ | H | H | H | |

* Nicht erfindungsgemäß

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Plasmopara viticola an Reben sowie
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden Können. Als Hilfsstoffe kommen dafür im wesentlichen in Frage: Lösungsmittel wie Aromaten (z.B. Xylol, chlorierte Aromaten (z.B. Chlorbenzole), Paraffine (z B. Erdölfraktionen), Alkohole (z.B. Methanol, Butanol) , Ketone (z.B. Cyclohexanon), Amine (z. B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew. % Wirkstoff.

Die Aufwandmengen liegen je nach Art des gewünschten Effektes zwischen 0,02 und 3 kg Wirkstoff oder mehr je ha. Die neuen Verbindungen Können auch im Materialschutz eingesetzt werden zum Beispiel gegen Paecilomyces variotii. Bei der Anwendung der Wirkstoffe im Materialschutz u.a. zur Bekämpfung holzzerstörender Pilze wie Coniophora puteana und Polystictus versicolor eingesetzt werden. Die neuen Wirkstoffe Können auch als fungizid wirksame Bestandteile öliger Holzschutzmittel zum Schutz von Holz gegen holzverfärbende Pilze, wie Aureobasidium pullulans, eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew. -Teile der Verbindung Nr. 2 mit 10 Gew.-Teilen N-Methyl-α-pyrrolidon und erhält eine Lösung, die zur Anwendung in Form Kleinster Tropfen geeignet ist.

II. 20 Gew.-Teile der Verbindung Nr. 23 werden in einer Mischung gelöst, die aus 80 Gew.-Teilen Xylol,

10 Gew.-Teilen des Anlagerungsproduktes von 8 bis 10 Mol Ethylenoxid an 1 Mol Ölsaure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 63 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 71 werden in einer Mischung gelöst. die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 75 werden mit 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 78 werden mit 97 Gew.-Teilen feinteiligem Kaolin innig vermischt. Man erhält auf diese Weise ein Stäubemittel, das 3 Gew.% des Wirkstoffs enthält.

VII.30 Gew.-Teile der Verbindung Nr. 102 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 142 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 148 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoffe wurden N-Benzyl-trans-4-tert.-butylcyclohexylamin (A) - bekannt aus J. Org. Chem. 48, 3412 (1983) und N-Tridecyl-2,6-dimethylmorpholin (B) - bekannt aus DE 11 64 152 und DE 11 73 722 - benutzt.

Anwendungsbeispiel 1

Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielt, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22 °C und 75 bis 80 % relativer Luftfeuchtigkeit aufgestellt. Nach 7 Tagen wurde das Ausmaß der Mehltauentwicklung ermittelt.

Bei diesem Versuch war mit 0,025%iger und 0,006 %iger Wirkstoffzubereitung der Verbindungen 2, 63, 71, 75, 78, 44 und 102 die Schadpilzentwicklung weitgehend zu verhindern, während die Vergleichswirkstoffe A und B starken Befall nicht verhinderten (unbehandelt Totalbefall).

Anwendungsbeispiel 2

Wirksamkeit gegen Gurkenmehltau (kurativ)

Blätter von in Töpfen gewachsenen Gurkenkeimlingen der Sorte "Chinesische Schlange" wurden im Zweiblattstadium mit einer wäßrigen Konidiensuspension des Gurkenmehltaus besprüht. Nach einem Tag wurden diese Pflanzen mit wäßriger Spritzbrühe, die 80 % Wirkstoff und 20 % Emulgiermittel in der Trockensubstanz enthielt, bis zur Tropfnässe besprüht und im Gewächshaus bei Temperaturen zwischen 20 und 22°C und 70 bis 80 % Luftfeuchtigkeit aufgestellt. 21 Tage nach der Inoculation wurde das Ausmaß des Pilzbefalles ermittelt.

Bei diesem Versuch war der Befall der Blätter nach Behandlung mit 0,025%iger Wirkstoffzubereitung der Verbindungen 2, 23, 26, 63, 71, 75, 78, 54, und 102 gering, während die Vergleichswirkstoffe A und B starken Befall nicht verhinderten (unbehandelt Totalbefall).

Anwendungsbeispiel 3

Wirksamkeit gegen Botrytis cinerea an Paprika

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Bei diesem Versuch war der Befall der Blätter nach Behandlung mit 0,05%iger Wirkstoffzubereitung bei den erfindungsgemäßen Verbindungen 14, 18, 22, 25, 26, 53, 66, 72, 82, 98, 100, 104, 105 und 131 gering, während bei den Vergleichswirkstoffen A und B Totalbefall auftrat.

Anwendungsbeispiel 4

Wirksamkeit gegen Septoria nodorum

Blätter von in Töpfen gewachsenen Weizenkeimlingen der Sorte "Jubilar" wurden mit wäßriger Spritzbrühe, die 80 % Wirkstoff und 20 % Emulgiermittel in der Trockensubstanz enthielt, bis zur Tropfnässe besprüht. Am folgenden Tag wurden die angetrockneten Pflanzen mit einer wäßrigen Sporensuspension von Septoria nodorum infiziert und dann für 7 Tage bei 17 bis 19°C und 95 % relativer Luftfeuchtigkeit weiter kultiviert. Das Ausmaß des Pilzbefalls wurde dann visuell ermittelt.

Bei diesem Versuch war der Befall der Blätter nach Behandlung mit 0,05%iger Wirkstoffzubereitung bei den erfindungsgemäßen Verbindungen 14, 18, 22, 25, 26, 53, 59, 66, 72, 82, 98, 100, 105 und 131 und gering, während bei den Vergleichswirkstoffen A und B starker Befall auftrat.

## Ansprüche

1. Cyclohexylamine der allgemeinen Formel I,

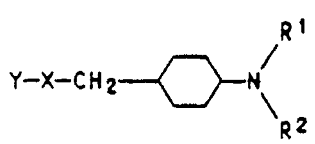

I

worin

| | |
|---|---|
| X | eine Einfachbindung oder eine ggf. durch eine oder mehrere Alkylgruppen mit jeweils 1 bis 5 C-Atomen substituierte Alkylen-Kette von 1 bis 12 C-Atomen bedeutet, in der ggf. 1 bis 4 C-Atome durch O, S oder N ersetzt sein können, |
| Y | einen Cyclohexyl-, 4-Cyclohexylcyclohexyl-, Perhydro-1- bzw.-2-naphthyl-, oder Piperidylrest bedeutet, die ggf. durch eine bis drei Alkyl-, Alkoxy- oder Alkylthiogruppen mit 1 bis 12 C-Atomen oder durch ein bis drei Halogen-, Hydroxy- oder Trifluormethylgruppen substituiert sein können, |
| $R^1$ und $R^2$ | gleich oder verschieden sind und unabhängig voneinander Wasserstoff, Cycloalkyl, Cycloalkenyl oder Arylalkyl-Reste mit bis zu 12 C-Atomen bedeuten, die ihrerseits ggf. wiederum durch ein bis drei Alkyl-, Alkenyl-, Alkinyl-, Alkoxy- oder Alkylthioreste mit 1 bis 4 C-Atomen oder Cyclohexyl oder 4-tert.-Butylcyclohexyl substituiert sein können oder |
| $R^1$ und $R^2$ | zusammen eine Alkylengruppe mit zwei bis sechs C-Atomen bedeuten und gemeinsam mit dem N-Atom einen Ring bilden, in dem ggf. bis zu drei C-Atome durch O, S oder N ersetzt sein können und der seinerseits durch ein bis drei Alkylreste mit 1 - 4 C-Atomen substituiert sein kann, |

sowie deren Säureadditionssalze, ausgenommen die Verbindungen in denen X eine Einfachbindung, Y Cyclohexyl, $R^1$ und $R^2$ Wasserstoff bedeuten.

2. Verfahren zur Herstellung der Cyclohexylamine der im Anspruch 1 angegebenen Formel I, dadurch gekennzeichnet, daß man Cyclohexylamine der Formel II

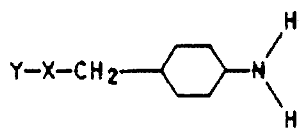

II

mit Verbindungen der Formeln III bzw. IV,

$R^1 - A$ 
$R^2 - A$ III

$R^1 - A$ 
$R^2 - A$ IV,

in denen $R^1$ und $R^2$ die im Anspruch 1 genannten Bedeutungen (außer Wasserstoff) haben und A für eine nucleophil verdrängbare Abgangsgruppe steht, umsetzt oder Verbindungen der Formel II mit Aldehyden oder Ketonen der Formel $R^8CHO$ oder $R^8$-CO-$R^9$ in Gegenwart eines Reduktionsmittels umsetzt, wobei $R^8$ und $R^9$ die gleichen Bedeutungen wie $R^1$ und $R^2$ jedoch mit einem C-Atom weniger haben.

3. Verfahren zur Herstellung von Cyclohexylaminem der im Anspruch 2 angegebenen Formel II, dadurch gekennzeichnet, daß man Arylnitroverbindungen der Formel V

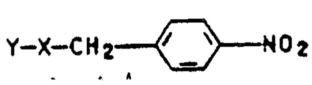

(V)

in der X und Y die im Anspruch 2 genannten Bedeutungen haben, nach bekannten Methoden zu entsprechenden Anilinen reduziert und diese in Gegenwart eines Katalysators mit Wasserstoff unter Druck hydriert.

4. Verfahren zur Herstellung von Zwischenprodukten der im Anspruch 3 angegebenen Formel V, dadurch gekennzeichnet, daß man

a) Verbindungen der Formel VI

$$Y{-}X{-}P^{\oplus}(C_6H_5)_3{-}Hal^{\ominus} \qquad VI$$

in der X und Y die im Anspruch 3 genannte Bedeutung haben und Hal für Chlorid oder Bromid steht,

mit 4-Nitrobenzaldehyd in Gegenwart einer Base und eines Lösungsmittels umsetzt, oder
b) Verbindungen der Formel VII,

$$Y-(CH=CH)_n-CHO \qquad VII$$

in der Y die im Anspruch 3 genannte Bedeutung hat und n die Werte 0 oder 1 annehmen kann, in Gegenwart einer Base mit 4-Nitrophenylalkylketonen umsetzt, oder
c ) Verbindungen der Formel X,

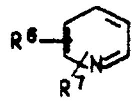

(X)

in der $R^6$ und $R^7$ die für Y definierten Substituenten des Piperidylrestes bedeuten, mit Verbindungen der Formel XI,

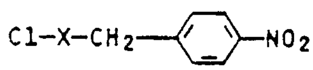

(XI)

in der X die oben genannte Bedeutung hat, in einem Lösungsmittel umsetzt und die Reaktionsprodukte hydriert.

**5.** Fungizid, enthaltend einen festen oder flüssigen Trägerstoff und ein Cyclohexylamin der allgemeinen Formel I

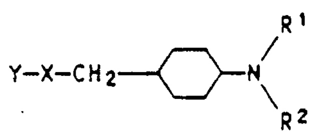

worin

| | |
|---|---|
| X | eine Einfachbindung oder eine ggf. durch eine oder mehrere Alkylgruppen mit jeweils 1 bis 5 C-Atomen substituierte Alkylen-Kette von 1 bis 12 C-Atomen bedeutet, in der ggf. 1 bis 4 C-Atome durch O, S oder N ersetzt sein können, |
| Y | einen Cyclohexyl-, 4-Cyclohexylcyclohexyl-, Perhydro-1- bzw.-2-naphthyl-, oder Piperidylrest bedeutet, die ggf. durch eine bis drei Alkyl-, Alkoxy-, Alkylthiogruppen mit 1 bis 12 C-Atomen oder durch ein bis drei Halogen-, Hydroxy- oder Trifluormethylgruppen substituiert sein können, |
| $R^1$ und $R^2$ | gleich oder verschieden sind und unabhängig voneinander Wasserstoff, Cycloalkyl, Cycloalkenyl oder Arylalkyl-Reste mit bis 12 C-Atomen bedeuten, die ihrerseits ggf. wiederum durch ein bis drei Alkyl-, Alkenyl-, Alkinyl-, Alkoxy- oder Alkylthioreste mit 1 bis 4 C-Atomen oder Cyclohexyl oder 4-tert.-Butylcyclohexyl substituiert sein können, oder |
| $R^1$ und $R^2$ | zusammen eine Alkylengruppe mit zwei bis sechs C-Atomen bedeuten und gemeinsam mit dem N-Atom einen Ring bilden, in dem ggf. bis zu drei C-Atome durch O, S oder N ersetzt sein können und der seinerseits durch ein bis drei Alkylreste mit 1 - 4 C-Atomen substituiert sein kann, |
| | sowie deren Säureadditionssalze, ausgenommen die Verbindungen in denen X eine Einfachbindung, Y Cyclohexyl, $R^1$ und $R^2$ Wasserstoff bedeuten. |

**6.** Verfahren zur Herstellung eines Fungizids, dadurch gekennzeichnet, daß man einen festen oder flüssigen Trägerstoff vermischt mit einer Verbindung gemäß Anspruch 1.

7. Verfahren zur Bekämpfung von Pilzen, dadurch gekennzeichnet, daß man die Pilze oder die vor Pilzbefall zu schützenden Materialien, Pflanzen, Boden oder Saatgüter behandelt mit einen Cyclohexylamin der allgemeinen Formel I

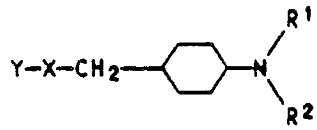

worin

X eine Einfachbindung oder eine ggf. durch eine oder mehrere Alkylgruppen mit jeweils 1 bis 5 C-Atomen substituierte Alkylen-Kette von 1 bis 12 C-Atomen bedeutet, in der ggf. 1 bis 4 C-Atome durch O, S oder N ersetzt sein können,

Y einen Cyclohexyl-, 4-Cyclohexylcyclohexyl-, Perhydro-1- bzw.-2-naphthyl-, oder Piperidylrest bedeutet, die ggf. durch eine bis drei Alkyl-, Alkoxy-, Alkylthiogruppen mit 1 bis 12 C-Atomen oder durch ein bis drei Halogen-, Hydroxy- oder Trifluormethylgruppen substituiert sein können,

$R^1$ und $R^2$ gleich oder verschieden sind und unabhängig voneinander Wasserstoff, Cycloalkyl, Cycloalkenyl oder Arylalkyl-Reste mit bis zu 12 C-Atomen bedeuten, die ihrerseits ggf. wiederum durch ein bis drei Alkyl-, Alkenyl-, Alkinyl-, Alkoxy- oder Alkylthioreste mit 1 bis 4 C-Atomen oder Cyclohexyl oder 4-tert.-Butylcyclohexyl substituiert sein können, oder

$R^1$ und $R^2$ zusammen eine Alkylengruppe mit zwei bis sechs C-Atomen bedeuten und gemeinsam mit dem N-Atom einen Ring bilden, in dem ggf. bis zu drei C-Atome durch O, S oder N ersetzt sein können und der seinerseits durch ein bis drei Alkylreste mit 1 - 4 C-Atomen substituiert sein kann,

sowie deren Säureadditionssalze, ausgenommen die Verbindungen in denen X eine Einfachbindung, Y Cyclohexyl, $R^1$ und $R^2$ Wasserstoff bedeuten.

8. Verbindung der Formel V

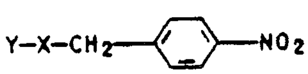

(V)

in der

X eine Einfachbindung oder eine ggf. durch eine oder mehrere Alkylgruppen mit jeweils 1 bis 5 C-Atomen substituierte Alkylen-Kette von 1 bis 12 C-Atomen bedeutet, in der ggf. 1 bis 4 C-Atome durch O, S oder N ersetzt sein können, und

Y einen Cyclohexyl-, 4-Cyclohexylcyclohexyl-, Perhydro-1- bzw. 2-naphthyl-, oder Piperidylrest bedeutet, die ggf. durch eine bis drei Alkyl-, Alkoxy- oder Alkylthiogruppen mit 1 bis 12 C-Atomen oder durch ein bis drei Halogen-, Hydroxy- oder Trifluormethylgruppen substituiert sein können, bedeuten.

9. Verbindung gemäß Anspruch 1, dadurch gekennzeichnet, daß X eine 1,2-Ethylengruppe, Y Cyclohexyl und $R^1$ und $R^2$ Wasserstoff sind.

10. Verbindung gemäß Anspruch 1, dadurch gekennzeichnet, daß X eine Methylen- oder 1,2-Ethylengruppe, Y Perhydro-1- oder -2-naphthyl und $R^1$ und $R^2$ Wasserstoff bedeuten.

## Claims

1. A cyclohexylamine of the formula

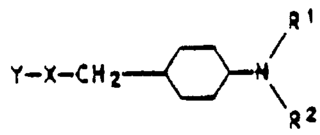

(I)

where X is a single bond or an alkylene chain of 1 to 12 carbon atoms which is unsubstituted or substituted by one or more alkyl groups, each of 1 to 5 carbon atoms, and in which 1 to 4 carbon atoms may be replaced by O, S or N. Y is cyclohexyl, 4-cyclohexylcyclohexyl, perhydro-1- or -2-naphthyl or piperidyl which may be substituted by one, two or three alkyl, alkoxy or alkylthio groups of 1 to 12 carbon atoms or by one, two or three halogen, hydroxyl or trifluoromethyl groups, and $R^1$ and $R^2$ are identical or different and independently of one another are each hydrogen, cycloalkyl, cycloalkenyl or aralkyl of up to 12 carbon atoms, which in turn may be substituted by one, two or three alkyl, alkenyl, alkynyl, alkoxy or alkylthio radicals of 1 to 4 carbon atoms or cyclohexyl or 4-tert-butylcyclohexyl, or $R^1$ and $R^2$ together form an alkylene group of 2 to 6 carbon atoms and, together with the N atom, form a ring in which up to three carbon atoms may be replaced by O, S or N and which in turn may have one, two or three alkyl substituents of 1 to 4 carbon atoms, and their addition salts with acids, with the exception of those compounds in which X is a single bond, Y is cyclohexyl and $R^1$ and $R^2$ are each hydrogen.

2. A process for preparing a cyclohexylamine of the formula I indicated in claim 1, which comprises reaching a cyclohexylamine of the formula II

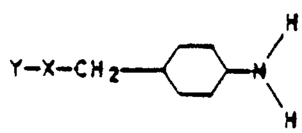

(II)

with a compound of the formula III or IV

$R^1$-A (III)
$R^2$-A

-$R^1$-A (IV)
-$R^2$-A

where $R^1$ and $R^2$ have the meanings given in claim 1 (except hydrogen) and A is a nucleophilically displaceable leaving group, or reacting a compound of the formula II with an aldehyde or a ketone of the formula $R^8$CHO or $R^8$-CO-$R^9$ in the presence of a reducing agent, where $R^8$ and $R^9$ are as defined for $R^1$ and $R^2$, but with one carbon atom less in each case.

3. A process for preparing a cyclohexylamine of the formula II as indicated in claim 2, which comprises reducing an arylnitro compound of the formula V

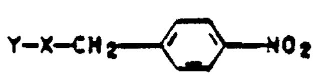

(V)

where X and Y are as defined in claim 2, by a known method to give the corresponding aniline, and then hydrogenating the latter using hydrogen under superatmospheric pressure in the presence of a catalyst.

4. A process for preparing an intermediate of the formula V as indicated in claim 3, which comprises a) reacting a compound of the formula VI

$$Y\text{-}X\text{-}P^{\oplus}(C_6H_5)_3\text{-}Hal^{\ominus} \qquad (VI)$$

where X and Y are as defined in claim 3 and Hal is chloride or bromide, with 4-nitrobenzaldehyde in solution and in the presence of a base, or b) reacting a compound of the formula VII

$$Y-(CH=CH)_n-CHO \qquad (VII)$$

where Y is as defined in claim 3 and n may be 0 or 1, with a 4-nitrophenyl alkyl ketone in the presence of a base, or c) reacting a compound of the formula X

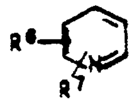

(X)

where $R^6$ and $R^7$ are each one of the substituents of the piperidyl radical defined for Y, with a compound of the formula XI

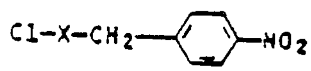

(XI)

where X is as defined above in solution, and hydrogenating the product.

5. A fungicide containing a solid or liquid carrier and a cyclohexylamine of the formula I

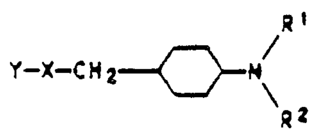

where X is a single bond or an alkylene chain of 1 to 12 carbon atoms which is unsubstituted or substituted by one or more alkyl groups, each of 1 to 5 carbon atoms, and in which 1 to 4 carbon atoms may be replaced by O, S or N. Y is cyclohexyl, 4-cyclohexylcyclohexyl, perhydro-1- or -2-naphthyl or piperidyl which may be substituted by one, two or three alkyl, alkoxy or alkylthio groups of 1 to 12 carbon atoms or by one, two or three halogen, hydroxyl or trifluoromethyl groups, and $R^1$ and $R^2$ are identical or different and independently of one another are each hydrogen, cycloalkyl, cycloalkenyl or aralkyl of up to 12 carbon atoms, which in turn may be substituted by one, two or three alkyl, alkenyl, alkynyl, alkoxy or alkylthio radicals of 1 to 4 carbon atoms or cyclohexyl or 4-tert-butylcyclohexyl, or $R^1$ and $R^2$ together form an alkylene group of 2 to 6 carbon atoms and, together with the N atom, form a ring in which up to three carbon atoms may be replaced by O, S or N and which in turn may have one, two or three alkyl substituents of 1 to 4 carbon atoms, and their addition salts with acids, with the exception of those compounds in which X is a single bond, Y is cyclohexyl and $R^1$ and $R^2$ are each hydrogen.

6. A process for preparing a fungicide, which comprises mixing a solid or liguid carrier with a compound as claimed in claim 1.

7. A process for combating fungi, wherein the fungi or the materials, plants, soil or seed to be protected against fungus attack are treated with a cyclohexylamine of the formula I

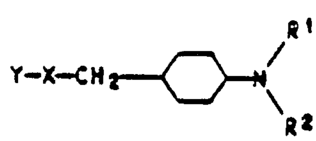

where X is a single bond or an alkylene chain of 1 to 12 carbon atoms, which is unsubstituted or substituted by one or more alkyl groups, each of 1 to 5 carbon atoms, and in which 1 to 4 carbon atoms may be replaced by O, S or N, Y is cyclohexyl, 4-cyclohexylcyclohexyl, perhydro-1- or -2-naphthyl or piperidyl which may be substituted by one, two or three alkyl, alkoxy or alkylthio groups of 1 to 12 carbon atoms or by one, two or three halogen, hydroxyl or trifluoromethyl groups, and $R^1$ and $R^2$ are identical or different and independently of one another are each hydrogen, cycloalkyl, cycloalkenyl or aralkyl of up to 12 carbon atoms, which in turn may be substituted by one, two or three alkyl, alkenyl, alkynyl, alkoxy or alkylthio radicals of 1 to 4 carbon atoms or cyclohexyl or 4-tert-butylcyclohexyl, or $R^1$ and $R^2$ together form an alkylene group of 2 to 6 carbon atoms and, together with the N atom, form a ring in which up to three carbon atoms may be replaced by O, S or N and which in turn may have one, two or three alkyl substituents of 1 to 4 carbon atoms, and their addition salts with acids, with the exception of those compounds in which X is a single bond, Y is cyclohexyl and $R^1$ and $R^2$ are each hydrogen.

**8.** A compound of the formula V

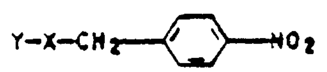

(V)

where X is a single bond or an alkylene chain of 1 to 12 carbon atoms which is unsubstituted or substituted by one or more alkyl groups of 1 to 5 carbon atoms, in which 1 to 4 carbon atoms may be replaced by O, S or N, and Y is cyclohexyl, 4-cyclohexylcyclohexyl, perhydro-1- or -2-naphthyl or piperidyl which may be substituted by one, two or three alkyl, alkoxy or alkylthio groups of 1 to 12 carbon atoms or by one, two or three halogen, hydroxyl or trifluoromethyl groups.

**9.** A compound as claimed in claim 1, where X is a 1,2-ethylene group, Y is cyclohexyl and $R^1$ and $R^2$ are hydrogen.

**10.** A compound as claimed in claim 1, where X is a methylene or 1,2-ethylene group, Y is perhydro-1- or -2-naphthyl and $R^1$ and $R^2$ are hydrogen.

## Revendications

**1.** Cyclohexylamines de formule générale I

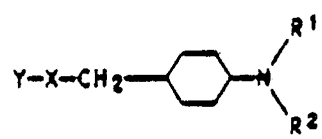

I,

dans laquelle

| | |
|---|---|
| X | représente une simple liaison ou une chaîne alkylène de 1 à 12 atomes C, éventuellement substituée par un ou plusieurs groupes alkyle ayant chacun 1 à 5 atomes C, dans laquelle 1 à 4 atomes C sont éventuellement remplacés par O, S ou N, |
| Y, | un reste cyclohexyle, 4-cyclohexylcyclohexyle, perhydro-1- ou -2-naphtyle ou pipéridyle, ces restes étant éventuellement substitués par un à trois groupes alkyle, alcoxy ou alkylthio de 1 à 12 atomes C ou par un à trois groupes halogéno-, hydroxy- ou trifluorométhyle, |
| $R^1$ et $R^2$ | sont identiques ou différents et représentent, indépendamment l'un de l'autre, hydrogène, cycloalkyle, cycloalcényle ou des restes arylalkyle ayant jusqu'à 12 atomes C, ces restes pouvant à leur tour être substitués par un à trois restes alkyle, alcényle, alcynyle, alcoxy ou alkylthio de 1 à 4 atomes C, ou par cyclohexyle ou 4-tert-butylcyclohexyle, ou |

$R^1$ et $R^2$ représentent, ensemble, un groupe alkylène à 2 à 6 atomes C et forment, avec l'atome N, un cycle dans lequel jusqu'à 3 atomes C peuvent être remplacés par O, S ou N ou qui, à son tour, peut être substitué par un à trois restes alkyle de 1 à 4 atomes C, ainsi que leurs sels d'addition d'acide, exceptés les composés dans lesquels X représente une simple liaison, Y, cyclohexyle, $R^1$ et $R^2$, hydrogène.

**2.** Procédé de préparation des cyclohexylamines de la formule I, indiquée dans la revendication 1, caractérisé par le fait que l'on fait réagir des cyclohexylamines de formule II

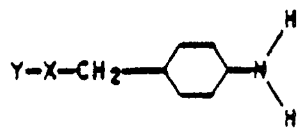

II

avec des composés des formules III ou IV,

$R^1$ - A

$R^2$ - A

III

-$R^1$ - A

-$R^2$ - A

IV

dans lesquelles $R^1$ et $R^2$ ont les significations données dans la revendication 1 (excepté hydrogène) et A est mis pour un groupe de départ déplaçable par un réactif nucléophile, ou on fait réagir, en présence d'un agent réducteur, des composés de formule II avec des aldéhydes ou des cétones de formule $R^8CHO$ ou $R^8$-CO-$R^9$, $R^8$ et $R^9$ ayant les mêmes significations que $R^1$ et $R^2$, mais avec un atome C en moins.

**3.** Procédé de préparation de cyclohexylamines de la formule II, indiquée dans la revendication 2, caractérisé par le fait que l'on réduit, selon des méthodes connues, des dérivés arylnitrés de formule V

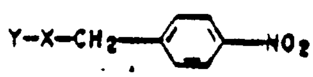

V,

dans laquelle X et Y ont les significations données dans la revendication 2, pour obtenir des anilines correspondantes et on hydrogène celles-ci, en présence d'un catalyseur, avec de l'hydrogène, sous pression.

**4.** Procédé de préparation de produits intermédiaires de la formule V, indiquée dans la revendication 3, caractérisé par le fait que l'on fait réagir
a) des composés de formule VI

$$Y\text{-}X\text{-}P^{\oplus}(C_6H_5)_3\text{-}Hal^{\ominus} \qquad VI,$$

dans laquelle X et Y ont les significations données dans la revendication 3 et Hal est mis pour chlorure ou bromure, avec du 4-nitrobenzaldéhyde, en présence d'une base et d'un solvant, ou
b) on fait réagir, en présence d'une base, des composés de formule VII

$$Y\text{-}(CH{=}CH)_n\text{ - }CHO \qquad VII,$$

dans laquelle Y a la signification donnée dans la revendication 3 et n peut signifier 0 ou 1, avec des 4-nitrophénylalkylcétones, ou

c) on fait réagir, dans un solvant, des composés de formule X,

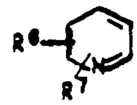

(X)

dans laquelle $R^6$ et $R^7$ représentent les substituants du reste pipéridyle définis pour Y, avec des composés de formule XI,

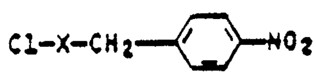

(XI),

dans laquelle X a la signification donnée plus haut, et on hydrogène les produits de réaction.

**5.** Fongicide, contenant un véhicule solide ou liquide et une cyclohexylamine de formule générale I

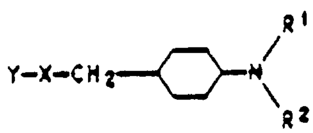

I,

dans laquelle

| | |
|---|---|
| X | représente une simple liaison ou une chaîne alkylène de 1 à 12 atomes C, éventuellement substituée par un ou plusieurs groupes alkyle ayant chacun 1 à 5 atomes C, dans laquelle 1 à 4 atomes C sont éventuellement remplacés par O, S ou N, |
| Y, | un reste cyclohexyle, 4-cyclohexylcyclohexyle, perhydro-1- ou -2-naphtyle ou pipéridyle, ces restes étant éventuellement substitués par un à trois groupes alkyle, alcoxy ou alkylthio de 1 à 12 atomes C ou par un à trois groupes halogéno-, hydroxy- ou trifluorométhyle, |
| $R^1$ et $R^2$ | sont identiques ou différents et représentent, indépendamment l'un de l'autre, hydrogène, cycloalkyle, cycloalcényle ou des restes arylalkyle ayant jusqu'à 12 atomes C, ces restes pouvant à leur tour être substitués par un à trois restes alkyle, alcényle, alcynyle, alcoxy ou alkylthio de 1 à 4 atomes C, ou par cyclohexyle ou 4-tert-butylcyclohexyle, ou |
| $R^1$ et $R^2$ | représentent, ensemble, un groupe alkylène à 2 à 6 atomes C et forment, avec l'atome N, un cycle dans lequel jusqu'à 3 atomes C peuvent être remplacés par O, S ou N ou qui, à son tour, peut être substitué par un à trois restes alkyle de 1 à 4 atomes C, ainsi que leurs sels d'addition d'acide, exceptés les composés dans lesquels X représente une simple liaison, Y, cyclohexyle, $R^1$ et $R^2$, hydrogène. |

**6.** Procédé de préparation d'un fongicide, caractérisé par le fait que l'on mélange un véhicule solide ou liquide avec un composé de la revendication 1.

**7.** Procédé de lutte contre les champignons, caractérisé par le fait que l'on traite les champignons ou les matériaux, plantes, sol ou semences à protéger contre l'attaque par les champignons avec une cyclohexylamine de formule générale I

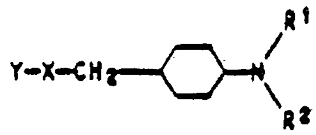

I,

dans laquelle

- X représente une simple liaison ou une chaîne alkylène de 1 à 12 atomes C, éventuellement substituée par un ou plusieurs groupes alkyle ayant chacun 1 à 5 atomes C, dans laquelle 1 à 4 atomes C sont éventuellement remplacés par O, S ou N,
- Y, un reste cyclohexyle, 4-cyclohexylcyclohexyle, perhydro-1- ou -2-naphtyle ou pipéridyle, ces restes étant éventuellement substitués par un à trois groupes alkyle, alcoxy ou alkylthio de 1 à 12 atomes C ou par un à trois groupes halogéno-, hydroxy- ou trifluorométhyle,
- $R^1$ et $R^2$ sont identiques ou différents et représentent, indépendamment l'un de l'autre, hydrogène, cycloalkyle, cycloalcényle ou des restes arylalkyle ayant jusqu'à 12 atomes C, ces restes pouvant à leur tour être substitués par un à trois restes alkyle, alcényle, alcynyle, alcoxy ou alkylthio de 1 à 4 atomes C, ou par cyclohexyle ou 4-tert-butylcyclohexyle, ou
- $R^1$ et $R^2$ représentent, ensemble, un groupe alkylène à 2 à 6 atomes C et forment, avec l'atome N, un cycle dans lequel jusqu'à 3 atomes C peuvent être remplacés par O, S ou N ou qui, à son tour, peut être substitué par un à trois restes alkyle de 1 à 4 atomes C, ainsi que leurs sels d'addition d'acide, exceptés les composés dans lesquels X représente une simple liaison, Y, cyclohexyle, $R^1$ et $R^2$, hydrogène.

**8.** Composé de formule V

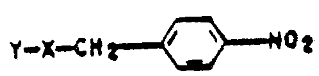

(V),

dans laquelle

- X représente une simple liaison ou une chaîne alkylène de 1 à 12 atomes C, éventuellement substituée par un ou plusieurs groupes alkyle ayant chacun 1 à 5 atomes C, dans laquelle 1 à 4 atomes C sont éventuellement remplacés par O, S ou N,
- Y, un reste cyclohexyle, 4-cyclohexylcyclohexyle, perhydro-1- ou -2-naphtyle ou pipéridyle, ces restes étant éventuellement substitués par un à trois groupes alkyle, alcoxy ou alkylthio de 1 à 12 atomes C ou par un à trois groupes halogéno-, hydroxy- ou trifluorométhyle.

**9.** Composé selon la revendication 1, caractérisé par le fait que X est un groupe 1,2-éthylène, Y est cyclohexyle et $R^1$ et $R^2$ sont hydrogène.

**10.** Composé selon la revendication 1, caractérisé par le fait que X représente un groupe méthylène ou 1,2-éthylène, Y, perhydro-1- ou -2-naphtyle et $R^1$ et $R^2$, hydrogène.